(19) Europäisches Patentamt
European Patent Office
Office européen des brevets

(11) Publication number: 0 323 624 A2

(12) # EUROPEAN PATENT APPLICATION

(21) Application number: 88121651.9

(51) Int. Cl.4: C07C 67/60 , C07C 69/14

(22) Date of filing: 24.12.88

(30) Priority: 06.01.88 US 141356

(43) Date of publication of application: 12.07.89 Bulletin 89/28

(84) Designated Contracting States: BE CH DE FR GB IT LI

(71) Applicant: BASF Corporation
9 Campus Drive
Parsippany, NJ 07054(US)

(72) Inventor: Thurman, Laurance R.
1207 Lake Street
Clute Texas 77531(US)
Inventor: Harris, James B.
4614 Northfork Drive
Pearland Texas 77584(US)
Inventor: Caffrey, Caren C.
110 Lake Road No. 716
Lake Jackson Texas 77566(US)

(74) Representative: Kinzel, Klaus, Dr. et al
BASF Aktiengesellschaft Patentabteilung
ZSP - C 6 Carl-Bosch-Strasse 38
D-6700 Ludwigshafen(DE)

(54) **Purifying carboxylic acid esters through use of selected reducing agents.**

(57) Carboxylic acid esters having low, storage-stable acid content and low storage-stable APHA color may be prepared by adding selected reducing agents, i.e. hydrazine, into the ester distillation train.

EP 0 323 624 A2

## PURIFYING CARBOXYLIC ACID ESTERS THROUGH USE OF SELECTED REDUCING AGENTS

The subject invention relates to the purification of carboxylic acid esters. More particularly, the subject invention relates to a process for the purification of alkanol esters of carboxylic acids through the incorporation of certain selected reducing agents prior to or during distillation.

Carboxylic acid esters are important commodity chemicals having a wide variety of uses, for example as solvents, plasticizers, and in the case of diesters, as intermediates for the production of polyesters. Such carboxylic acid esters are readily prepared by the esterification of mono-, di-, or polycarboxylic acids with alcohols, generally in the presence of strongly acid catalysts. Water produced during the esterification reaction is usually removed simultaneously, often by simple distillation, but occasionally by azeotropic distillation employing a suitable azeotroping solvent. Following neutralization of the catalyst, the product ester is then purified by fractional distillation employing one or more stages depending upon the product purity desired.

When relatively pure carboxylic acids and relatively pure alcohols are available as feedstocks, a relatively pure product with low color and low residual acid content, and which maintains these desirable characteristics during storage, may readily be obtained. However, it is frequently desirable to manufacture esters from crude feedstocks which are the by-product streams derived from production of other basic chemicals. Such by-product streams are found, for example, in the manufacture of oxo process alcohols; in the production of diols such as ethylene glycol, propylene glycol, butylene glycol; in the manufacture of cyclics such as tetrahydrofuran; and in the manufacture of oxygenated hydrocarbons such as cyclohexanone, cyclohexanol, adipic acid, caprolactone, and the like. The use of alcohols derived from process streams such as these is desirable economically as the result is a product containing chemical values which otherwise would be sold at low prices or would have to be disposed of by burning, deep well injection, or the like.

Unfortunately, such use has been problematic in the past, as the ester product contains numerous impurities, particularly those of an acid nature. Additionally, these products have a high APHA color even after multiple distillations. Finally, the acidity and color of these products may be unstable, rapidly increasing with the passage of time.

In U.S. patent 3,818,071, crude esters such as the dialkylphthalates are treated with sodium carbonate and steam at temperatures in excess of 100 °C to produce a product having lower acidity. However this process requires specialized equipment and thus is not suitable for many existing facilities. U.S. patent 3,891,694 utilizes steam treating an ester in the presence of an aliphatic or aromatic peracid at temperatures greater than 100 °C. However peracids are powerful oxidizing agents, and thus their use in conjunction with flammable organic esters leads to safety problems which are undesirable in modern chemical processing. Furthermore, this method cannot be used with unsaturated esters which may polymerize at elevated temperatures in the presence of peroxygen compounds.

U.S. Patent 4,216,337 discloses a process for purifying esters by distilling off unreacted alcohol from the crude ester product, heating the ester so-obtained at a temperature of from 160 °C to 340 °C for 0.1 to 3 hours, contacting the heat-treated ester with an adsorbent at a temperature of from 30 °C to 150 °C, and finally separating the desired ester by distillation. This process is complex, requiring the use of substantial quantities of adsorbent as well as specialized apparatus. Additionally, heat treating esters at relatively high temperatures is known to increase the amount of high boiling impurities. While these impurities are easily removed during distillation, their formation represents a loss of product.

It has now been unexpectedly discovered that the color and acid impurity level found in carboxylic acid esters may both be improved through the addition of certain reducing agents prior to or during distillation of such esters. In addition, products so treated tend to retain low acidity and color upon storage.

Figure 1 represents a typical batch ester process utilizing a single esterification reactor and multiple distillation columns for purifying the ester product.

Carboxylic acid esters produced by this process may be mono-, di-, or polyesters. In the specification which follows, these mono-, di-, and polyester products will be referred to as "carboxylic acid esters" or simply "esters." Such esters are routinely produced by reaction of the appropriate mono-, di-, or poly carboxylic acid with the appropriate mono-, di-, or polyfunctional alcohol. When both the carboxylic acid and the alcohol are di- or polyfunctional, polyester oligomers and high molecular weight polyester polymers are produced. The term "polyester" as used herein does not contemplate such polymers, which contain repeating ester linkages, but only relatively low molecular weight products containing multiple ester groups. Thus the subject invention esters may be derived by reacting monocarboxylic acids with mono-, di-, or polyfunctional alcohols; or by reacting monofunctional alcohols with mono-, di-, or polycarboxylic acids.

The carboxylic acids useful in the process of the subject invention may be saturated, unsaturated, or aromatic carboxylic acids. Examples of saturated carboxylic acids include the branched and linear aliphatic carboxylic acids having the general formula

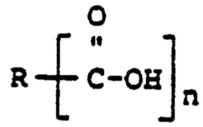

wherein R is an aliphatic, cycloaliphatic, or arylaliphatic radical having a valence n where n is a whole number from 1 to about 4, preferably 1 to 2. Preferred saturated aliphatic acids are acetic acid, proprionic acid, butyric acid, pentanoic acid, hexanoic acid, pivalic acid, benzylic acid, cyclohexane carboxylic acid, butanedioic acid, hexanedioic acid, and decanedioic acid.

Unsaturated aliphatic carboxylic acids have the same formula as presented above, but the organic radical must contain one or more ethylenic or acetylenic bonds. Examples are acrylic acid, methacrylic acid, butenoic acid, 1,4-butenedioic acid, cyclohexene carboxylic acid, 1,4-butynedioic acid, and the like. Aromatic carboxylic acids include benzoic acid, the terephthalic acids, and the like.

The alcohols useful in the process of the subject invention include the saturated and unsaturated aliphatic alcohols. Examples of such alcohols include methanol, ethanol, propanol, isopropanol, n-butanol, isobutanol, t-butanol, the various amyl alcohols, hexanol, ethylene glycol, propylene glycol, 1,4-butanediol, 1,4-butenediol, 1,4-butynediol, neopentyl glycol, 1,5-pentanediol, 2,2,4-trimethylpentan-1,5-diol, 1,6-hexanediol, 2-ethylhexanol, n-octanol, glycerine, cyclopentenol, cyclohexenol, cyclohexanol, cyclopentanol, benzyl alcohol, and the like.

Preferably used in the process of the subject invention are alcohol containing mixtures which are the by-product of various process streams. Such mixtures generally contain one or more alcohols, various saturated and unsaturated hydrocarbons, aldehydes, ketones, acids, and water. Such process streams may be used as such, may be concentrated by distillation, for example to remove water and various lights impurities, or may be chemically modified to vary their composition. Mixtures containing various amyl alcohols and their isomers are particularly useful. The wide compositional range of such mixtures is reflected in the examples.

The esterification process itself is completely conventional, as delineated with reference to figure 1. In figure 1, the carboxylic acid and alcohol feed streams S-1 and S-2 respectively, are charged to reactor R-1 along with the desired catalyst, generally concentrated sulfuric acid. Reactor R-1 is maintained at reflux, and lights, which include both saturated and unsaturated lower alkanes and cycloalkanes are taken off overhead. The R-1 take-off stream S-3, containing the ester product, water, unreacted acids and alcohols, and various impurities, is routed to neutralizing kettle K-1 where caustic is added to neutralize the acid catalyst. The neutralized crude ester stream is then routed via stream S-4 to the distillation train, in this case represented by distillation columns D-1 and D-2. The actual details of the distillation train will depend on the particular alcohol used and the ester thusly produced. The makeup of the input stream with regard to the various impurities will also affect the distillation train interconnections. Such process design parameters are easily determined by one skilled in the art.

In the process of the subject invention, certain specific reducing agents, to be defined hereinafter, are added to the ester while in the distillation train. Although the reducing agent may be added to the final distillation column in the train, it has been determined that the most appropriate location for injection of the reducing agent in at the input to the penultimate distillation column. This is particularly true when the reducing agent is supplied as an aqueous solution as this location favors facile elimination of the water thus added.

The specific reducing agents which have been found useful in reducing the concentration of the impurities responsible for increasing the acid content and color of esters in the process of the subject invention are hydrazine, hydroxylamine, and their salts. Examples of such salts are hydrazine sulfate, hydrazine hydrate, hydrazine hydrochloride, hydroxylamine hydrochloride, and the like. Preferably, these compounds are injected into the ester stream in the form of their aqueous solutions, particularly when the reducing agent is hydrazine, the most preferred embodiment.

The quantity of reducing agent required will vary depending upon the nature of the various carboxylic acid and/or alcohol feedstocks; the process parameters, particularly the processing temperatures; and the specifications of the ester product. When the reducing agents are injected as 35 weight percent aqueous solution based on hydrazine or hydroxylamine, generally from 50-900 ml of reducing agent solution, and

preferably from 200-400 ml of solution per hundred pounds of ester product will be utilized. These amounts correspond to about .5 g-mole to 9 g-moles and about 2 g-moles to about 4 g-moles of reducing agent per hundred pounds of product respectively. However lesser or greater amounts might be required in any given specific application. The amount is easily determined through routine procedures in, for example, a pilot plant or laboratory scale mock-up of the actual plant layout. In the claims, the term "effective amount" with regard to the reducing agent represents an amount of reducing agent which is effective to reduce the concentration of the impurities which cause the acid content, APHA color, and generally both the acid content and color to increase over time as compared to the increase in acid content and/or color of product produced in a similar process conducted without the addition of the reducing agent.

The invention will now be illustrated through reference to the following examples. These examples should not be viewed as limiting the scope of the invention in any way.

## Examples 1-2

In these examples, two co-product streams containing substantial quantities of n-amyl alcohol were utilized to make n-amylacetate. The esterification reaction was performed in a batch ester plant whose layout is similar to that in Figure 1 except that the R-1 take-off stream S-3 is routed to a second reactor for further reaction prior to neutralization. Concentrated sulfuric acid was used as the esterification catalyst. The distillation train consists of two distillation columns, with the ester product taken as the overhead from the second column.

The compositions of the two co-product streams, to the nearest percent by weight, are given below in Table I.

**Table I**

| Component | Stream: | CP-1 | CP-2 |
|---|---|---|---|
| cyclopentene | | <1 | <1 |
| cyclohexene | | 3 | <1 |
| n-amyl alcohol | | 36 | 72 |
| cycloamyl alcohol | | 3 | 13 |
| hexanal | | 14 | 1 |
| cyclohexanol | | 4 | 1 |
| cyclohexanone | | 1 | 1 |
| amyl acetate | | 1 | 0 |
| other | | 37 | 10 |
| | Total | 100 | 100 |

Following esterification but prior to neutralization, the crude ester take-off streams from the second reactor, E-1 and E-2, derived from CP-1, and CP-2 respectively, had the compositions presented in Table II.

Table II

| Component | Crude Ester Take-Off: | E-1 | E-2 |
|---|---|---|---|
| cyclopentene | | 0 | 1 |
| acetic acid | | <1 | 1 |
| n-amyl alcohol | | 3 | 4 |
| cycloamyl alcohol | | <1 | <1 |
| hexanal | | 1 | 1 |
| cyclohexanone | | 2 | 2 |
| n-amyl acetate | | 69 | 76 |
| cycloamyl acetate | | 7 | 8 |
| other | | 16 | 6 |
| | Total | 100 | 100 |

To determine the effect of added hydrazine, a portion of the E-2 stream was treated with hydrazine. Prior to the final distillation, 35 percent by weight aqueous hydrazine was injected into the penultimate distillation column input stream at a rate corresponding to about 100 ml hydrazine solution for each 35 pounds of final ester product. In each case, the final product contained approximately 86 percent by weight of n-amyl acetate and approximately 10 percent by weight of cycloamylacetate. Table III summarizes the acidity and color of the stored products.

Table III

| Crude Stream | Finished Ester Properties | |
|---|---|---|
| | Acid content[1] | APHA color |
| E-1 | 0.25 | >20 |
| E-1[2] | 0.11 | 5 |
| E-2 (hydrazine treated) | 0.003 | 5 |

[1]acid content as acetic acid in wt. %
[2]following an additional distillation

Table III indicates that the hydrazine treated ester contains a much lower acid content than could otherwise be achieved even after additional distillation. Furthermore, the color of the finished product is acceptable, and comparable to that achieved through additional distillation. Avoiding an additional distillation increases throughput and lowers the manufacturing cost of the ester product. The color of the hydrazine treated product also shows stability equal to that of the product purified through additional distillation.

## Claims

1. A process for reducing and stabilizing the acid content, APHA color, or both, in a carboxylic acid ester product prepared by the esterification of one or more carboxylic acids with one or more aliphatic alcohols, followed by purification in a distillation train containing one or more distillation columns, comprising: adding to the ester product before its entry into or during its residence in the distillation train, an effective amount of a reducing agent selected from the group consisting of hydrazine, hydroxylamine, their salts, and mixtures thereof.

2. The process of claim 1 wherein said distillation train contains at least two distillation columns and wherein said reducing agent is added to the penultimate distillation column of the distillation train.

3. The process of claim 1 wherein said reducing agent is supplied to the process in the form of an aqueous solution.

4. The process of claim 3 wherein said reducing agent is supplied to the process in an amount of from about 0.5 gram mole to about 9 gram moles per 100 pounds of ester product.

5. The process of claim 3 wherein said reducing agent is supplied to the process in an amount of from 0.5 gram mole to about 9 gram moles per 100 pounds of product.

6. A process for reducing and stabilizing the acid content, APHA color, or both, in a carboxylic acid ester product prepared by the esterification of one or more carboxylic acids with one or more aliphatic alcohols, followed by purification in a distillation train containing two or more distillation columns, comprising: adding to the ester product while still in the distillation train, an effective amount of hydrazine.

7. The process of claim 6 wherein said hydrazine is added to the penultimate distillation column of the distillation train.

8. The process of claim 6 wherein said hydrazine is supplied to the process in the form of an aqueous solution.

9. The process of claim 6 wherein said hydrazine is supplied to the process in an amount of from about 0.5 gram mole to about 9 gram moles per 100 pounds of ester product.

10. The process of clam 6 wherein said hydrazine is supplied to the process in an amount of from 2 gram moles to about 4 gram moles per 100 pounds of ester product.

11. A carboxylic acid ester having low acid content and low APHA color when prepared by the process of claim 1.

12. A carboxylic acid ester having low acid content and low APHA color when prepared by the process of claim 6.

O.Z. 2063/02486

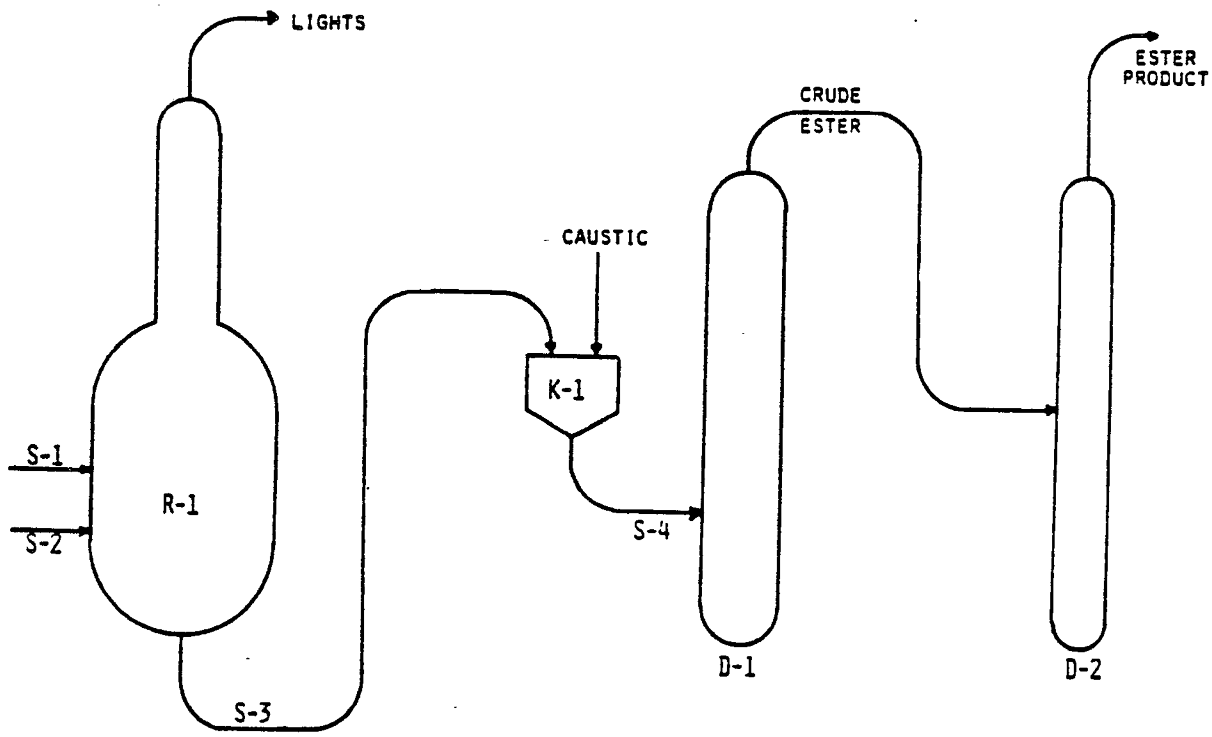


FIGURE 1